# EUROPEAN PATENT SPECIFICATION

(11) **EP 3 369 442 B1**
(45) Date of publication and mention of the grant of the patent: **16.03.2022**
(21) Application number: 16859960.3
(22) Date of filing: 28.10.2016
(51) Int. Cl.: A61M 1/16

(54) **BLOOD PURIFICATION APPARATUS**
BLUTREINIGUNGSVORRICHTUNG
DISPOSITIF DE PURIFICATION DU SANG

(30) Priority: 30.10.2015 JP 2015214221
(43) Date of publication of application: 05.09.2018
(73) Proprietor: Nikkiso Company Limited, Tokyo 150-6022 (JP)
(72) Inventor: MOCHIZUKI, Hiroaki, Makinohara-shi Shizuoka 421-0496 (JP); MATSUO, Sumiaki, Makinohara-shi Shizuoka 421-0496 (JP)
(74) Representative: von Hirschhausen, Helge
(86) International application number: PCT/JP2016/082076
(87) International publication number: WO 2017/073732

(56) References cited:
- EP-A2- 0 097 432
- JP-A- H05 208 048
- JP-A- 2007 285 830
- JP-A- 2014 513 990
- JP-B2- 3 714 947
- US-A- 5 366 630
- US-A1- 2002 032 403

## Description

### Technical Field

The present invention relates to a blood purification apparatus for purifying the blood of a patient while extracorporeally circulating the blood.

### Background Art

In general, a blood purification apparatus for giving dialysis treatment includes an arterial blood circuit and a venous blood circuit that form a blood circuit for extracorporeally circulating the blood of a patient, a blood purifier for purifying the blood that is extracorporeally circulated through the blood circuit, and an apparatus body provided with various treatment devices such as a blood pump for causing the blood circuit and the blood purifier to perform blood purification treatment. Vascular access catheters or puncture needles (an arterial puncture needle and a venous puncture needle) are attachable to the distal ends of the arterial blood circuit and the venous blood circuit, respectively.

For example, when the blood pump is activated after the arterial puncture needle and the venous puncture needle are stuck into the patient, the blood of the patient flows into the arterial blood circuit and the venous blood circuit. While the blood flows through the blood circuit, the blood is purified by the blood purifier. In the dialysis treatment, a dialysate introduction line for introducing dialysate into the blood purifier and a dialysate drain line for draining waste liquid from the blood purifier are connected to the blood purifier.

In such a known blood purification apparatus, a dialysate storage bag that stores dialysate to be introduced into the blood purifier is connected to the distal end of the dialysate introduction line, and a waste-liquid storage bag that stores waste liquid to be drained from the blood purifier is connected to the distal end of the dialysate drain line. The dialysate introduction line and the dialysate drain line are provided with a dialysate pump and a waste-liquid pump, respectively, each being a peristaltic pump. At the time of blood purification treatment, the dialysate pump and the waste-liquid pump are activated to rotate, whereby the dialysate stored in the dialysate storage bag is introduced into the blood purifier while the waste-liquid drained from the blood purifier is stored in the waste-liquid storage bag.

The dialysate storage bag and the waste-liquid storage bag are attached to respective weighing machines (a dialysate-side weighing machine and a waste-liquid-side weighing machine). While the dialysate pump and the waste-liquid pump are rotated, changes in the values measured by the respective weighing machines are monitored. In such a configuration, the change (actual value) in the weight caused by the rotation of each of the substitution pump and the waste-liquid pump is compared with the theoretical value corresponding thereto, whereby any difference between the two values (any error in the amount of discharge) can be detected. If it is determined that there is an error, the rotation speed of a corresponding one of the substitution pump and the waste-liquid pump can be corrected automatically. Patent literature documents US 5366630 A and JP 3714947 B2 both disclose similar blood purification apparatuses employing such a technique.

### Summary of Invention

### Technical Problem

The above known blood purification apparatus requires two weighing machines (the dialysate-side weighing machine and the waste-liquid-side weighing machine) for measuring the respective weights of the dialysate storage bag and the waste-liquid storage bag. That is, the manufacturing cost increases with the number of weighing machines. Moreover, if the two weighing machines have respective errors, the total error may be difficult to correct accurately.

Accordingly, both the dialysate storage bag and the waste-liquid storage bag may be weighed with a single (shared) weighing machine so that the cost is suppressed while the correction is performed accurately. In such a case, it is possible to detect any error in the rotation of the dialysate pump or the waste-liquid pump by comparing the value measured by the weighing machine and the theoretical value corresponding thereto. However, which of the pumps has that error cannot be determined. For example, if it is determined in accordance with the value measured by the weighing machine that either of the dialysate pump and the waste-liquid pump has an error, the waste-liquid pump may be corrected while the dialysate pump is regarded as not having any error. In such a case, however, if the dialysate pump actually has an error, the flow rate of the dialysate to be introduced into the blood purifier may have an error, giving adverse influence on the blood purification treatment.

The present invention has been conceived in view of the above circumstances and provides a blood purification apparatus capable of accurately correcting the rotation speeds of a dialysate pump and a waste-liquid pump with a reduced cost realized by a single weighing machine that weighs both a dialysate storage bag and a waste-liquid storage bag, the apparatus also being capable of reducing the error in the flow rate of dialysate to be introduced into a blood purification device.

### Solution to Problem

According to the invention of Claim 1, there is provided a blood purification apparatus that includes a blood circuit including an arterial blood circuit and a venous blood circuit and being capable of extracorporeally circulating blood of a patient from a distal end of the arterial blood circuit to a distal end of the venous blood circuit; a blood purification device interposed between the arterial blood circuit and the venous blood circuit of the blood circuit and being capable of purifying the blood flowing in the blood circuit; a dialysate introduction line for introducing dialysate into the blood purification device, and a dialysate drain line for draining waste liquid from the blood purification device; a dialysate pump in a form of a peristaltic pump provided to the dialysate introduction line, the dialysate pump being capable of causing the dialysate in the dialysate introduction line to flow when the dialysate pump is activated to rotate; a dialysate storage bag connected to a distal end of the dialysate introduction line and that stores the dialysate to be introduced into the blood purification device; a waste-liquid pump in a form of a peristaltic pump provided to the dialysate drain line, the waste-liquid pump being capable of causing the waste liquid in the dialysate drain line to flow when the waste-liquid pump is activated to rotate; a waste-liquid storage bag connected to a distal end of the dialysate drain line and that stores the waste liquid drained from the blood purification device; a single weighing machine capable of measuring a total weight of the dialysate storage bag and the waste-liquid storage bag; and a control unit capable of controlling the dialysate pump and the waste-liquid pump in accordance with a value measured by the weighing machine.

The control unit is configured to calculate a correction value for a rotation speed of the dialysate pump or for a value associated with the rotation speed in accordance with a change in the value measured by the weighing machine in a process of activating the dialysate pump such that the dialysate in the dialysate storage bag is caused to flow without being stored in the waste-liquid storage bag. The blood purification apparatus is characterized in that the control unit is configured to execute a correcting process in which the rotation speed of the dialysate pump or the value associated with the rotation speed is corrected in accordance with the calculated correction value and in which the control unit activates the dialysate pump to rotate while keeping the waste-liquid pump stopped.

According to the invention of Claim 2, the blood purification apparatus according to claim 1 is characterized in that the dialysate in the dialysate storage bag is allowed to flow into the venous blood circuit through the blood purification device.

According to the invention of Claim 3, the blood purification apparatus according to claim 1 is characterized by further including a substitution line that allows a predetermined portion of the venous blood circuit and the dialysate introduction line to communicate with each other. The apparatus is also characterized in that, in the correcting process, the dialysate in the dialysate storage bag is allowed to flow into the venous blood circuit through the substitution line.

According to the invention of Claim 4, the blood purification apparatus according to claim 2 or 3 is characterized in that, in the correcting process, a storage bag capable of storing liquid is connected to the distal end of the venous blood circuit.

According to the invention of Claim 5, the blood purification apparatus according to claim 1 is characterized in that the dialysate introduction line is provided with a flexible storage member that is capable of storing the dialysate flowing in the dialysate introduction line; and in that, in the correcting process, the dialysate in the dialysate storage bag is allowed to be stored in the storage member.

According to the invention of Claim 6, the blood purification apparatus according to claim 5 is characterized in that, in the correcting process, the control unit is configured to cause the liquid in the storage member to be discharged before activating the dialysate pump to rotate, and is configured to secure a capacity for storing the liquid that is to flow with an activation of the dialysate pump.

According to the invention of Claim 7, the blood purification apparatus according to claim 5 or 6 is characterized in that the storage member is a heating bag for heating the dialysate flowing in the dialysate introduction line in blood purification treatment.

According to the invention of Claim 8, the blood purification apparatus according to any of claims 1 to 7 is characterized in that the correcting process is performed before blood purification treatment.

According to the invention of Claim 9, the blood purification apparatus according to any of claims 1 to 7 is characterized in that the correcting process is performed during blood purification treatment.

According to the invention of Claim 10, the blood purification apparatus according to any of claims 1 to 9 is characterized in that, in the correcting process, the control unit corrects and controls the rotation speed of the dialysate pump in accordance with an error between a value measured by the weighing machine and a theoretical value.

According to the invention of Claim 11, the blood purification apparatus according to any of claims 1 to 10 is characterized in that, in the correcting process, the control unit is configured to activate the dialysate pump at a plurality of rotation speeds and setting correction values corresponding to the respective rotation speeds of the dialysate pump in accordance with changes in the values measured by the weighing machine that are obtained at the respective rotation speeds.

According to the invention of Claim 12, the blood purification apparatus according to any of claims 1 to 10 is characterized in that characteristic data on a rate of change in a flow rate with respect to a total number of revolutions of the dialysate pump is stored in advance; and in that, in the correcting process, the control unit is configured to set a correction value corresponding to the rotation speed of the dialysate pump in accordance with the characteristic data.

### Advantageous Effects of Invention

According to the invention of claim 1, the control unit is capable of calculating the correction value for the rotation speed of the dialysate pump or for the value associated with the rotation speed in accordance with the change in the value measured by the weighing machine in the process of activating the dialysate pump such that the dialysate in the dialysate storage bag is caused to flow without being stored in the waste-liquid storage bag. Therefore, the rotation speeds of the dialysate pump and the waste-liquid pump can be corrected accurately with a reduced cost realized by a single weighing machine that weighs both the dialysate storage bag and the waste-liquid storage bag. Furthermore, the error in the flow rate of the dialysate to be introduced into the blood purification device can be reduced. Furthermore, in the correcting process, the control unit activates the dialysate pump to rotate while keeping the waste-liquid pump stopped. Therefore, the dialysate flowing from the dialysate storage bag in the correcting process can be assuredly prevented from being stored in the waste-liquid storage bag.

According to the invention of Claim 2, the dialysate in the dialysate storage bag is allowed to flow into the venous blood circuit through the blood purification device. Therefore, the dialysate in the dialysate storage bag can be caused to flow into the venous blood circuit without providing any additional dedicated flow route.

According to the invention of Claim 3, the blood purification apparatus further includes the substitution line that allows the predetermined portion of the venous blood circuit and the dialysate introduction line to communicate with each other. Furthermore, in the correcting process, the dialysate in the dialysate storage bag is allowed to flow into the venous blood circuit through the substitution line. Therefore, the dialysate can be caused to flow into the blood circuit while detouring the blood purification device.

According to the invention of Claim 4, in the correcting process, the storage bag capable of storing liquid is connected to the distal end of the venous blood circuit. Therefore, the liquid flowing in the venous blood circuit with the activation of the dialysate pump can be prevented from spilling from the distal end of the venous blood circuit.

According to the invention of Claim 5, the dialysate introduction line is provided with the flexible storage member that is capable of storing the dialysate flowing in the dialysate introduction line. Furthermore, in the correcting process, the dialysate in the dialysate storage bag is allowed to be stored in the storage member. Therefore, the liquid that is caused to flow with the activation of the dialysate pump can be prevented from flowing into the blood circuit. Furthermore, since the storage member is flexible, the storage member is inflatable in accordance with the flow rate of the liquid that is caused to flow with the activation of the dialysate pump. Hence, the occurrence of resistance (flow resistance) at the flowing of the liquid can be suppressed, and the rotation speed of the dialysate pump can be corrected more accurately.

According to the invention of Claim 6, in the correcting process, the control unit is capable of causing the liquid in the storage member to be discharged before activating the dialysate pump to rotate, and is capable of securing the capacity for storing the liquid that is to flow with the activation of the dialysate pump. Therefore, the liquid that is caused to flow with the activation of the dialysate pump can be more assuredly stored in the storage member.

According to the invention of Claim 7, the storage member is the heating bag for heating the dialysate flowing in the dialysate introduction line in the blood purification treatment. Therefore, the liquid that is caused to flow with the activation of the dialysate pump can be stored by utilizing the heating bag, which is necessary for the blood purification treatment.

According to the invention of Claim 8, the correcting process is performed before blood purification treatment. Therefore, the blood purification treatment can be started immediately thereafter.

According to the invention of Claim 9, the correcting process is performed during the blood purification treatment. Therefore, even if any change occurs in the characteristic of the dialysate pump during the blood purification treatment, the rotation speed of the dialysate pump can be corrected.

According to the invention of Claim 10, in the correcting process, the control unit corrects and controls the rotation speed of the dialysate pump in accordance with the error between the value measured by the weighing machine and the theoretical value. Therefore, the rotation speed of the dialysate pump can be corrected more accurately, and the flow-rate error attributed to the rotation of the dialysate pump can be made smaller.

According to the invention of Claim 11, in the correcting process, the control unit is capable of activating the dialysate pump at a plurality of rotation speeds and setting the correction values corresponding to the respective rotation speeds of the dialysate pump in accordance with the changes in the values measured by the weighing machine that are obtained at the respective rotation speeds. Therefore, the flow-rate error attributed to the rotation of the dialysate pump can be made much smaller.

According to the invention of Claim 12, the characteristic data on the rate of change in the flow rate with respect to the total number of revolutions of the dialysate pump is stored in advance. Furthermore, in the correcting process, the control unit is capable of setting the correction value corresponding to the rotation speed of the dialysate pump in accordance with the characteristic data. Therefore, even if any flow-rate error occurs because of the aging of the dialysate pump, the control unit can set a correction value corresponding to the rotation speed of the dialysate pump more smoothly without activating the dialysate pump at a plurality of rotation speeds.

### Brief Description of Drawings

[Fig. 1] Fig. 1 is a schematic diagram illustrating a blood purification apparatus according to an embodiment of the present invention.
[Fig. 2] Fig. 2 is a schematic view illustrating a heating bag applied to the blood purification apparatus.
[Fig. 3] Fig. 3 is a perspective view of a heating device (with a lid portion thereof open) included in the blood purification apparatus.
[Fig. 4] Fig. 4 is a perspective view of the heating device with the heating bag attached thereto.
[Fig. 5] Fig. 5 is a perspective view of the heating device with the heating bag attached thereto and the lid portion thereof closed.
[Fig. 6] Fig. 6 is a schematic view illustrating a peristaltic pump applied to the blood purification apparatus.
[Fig. 7] Fig. 7 is a schematic diagram illustrating a state of a blood purification apparatus according to a first embodiment of the present invention in a correcting process.
[Fig. 8] Fig. 8 is a schematic diagram illustrating a state of a blood purification apparatus according to another embodiment of the present invention (in which dialysate is caused to flow into a venous blood circuit via a dialyzer) in a correcting process.
[Fig. 9] Fig. 9 is a flow chart of a control operation performed by a control unit included in the blood purification apparatus.
[Fig. 10] Fig. 10 is a flow chart of another control operation performed by the control unit.
[Fig. 11] Fig. 11 is a flow chart of a control operation performed by a control unit included in a blood purification apparatus according to another embodiment.
[Fig. 12] Fig. 12 is a flow chart of another control operation performed by the control unit.
[Fig. 13] Fig. 13 is a flow chart of a control operation performed by a control unit included in a blood purification apparatus according to yet another embodiment.
[Fig. 14] Fig. 14 is a flow chart of another control operation performed by the control unit.
[Fig. 15] Fig. 15 is a graph illustrating data on a characteristic of a dialysate pump that is to be referred to by the control unit.
[Fig. 16] Fig. 16 is a schematic diagram illustrating a state of a blood purification apparatus according to a second embodiment of the present invention in a correcting process.
[Fig. 17] Fig. 17 is a schematic diagram illustrating another state of the blood purification apparatus in the correcting process.

### Description of Embodiments

Embodiments of the present invention will now be described specifically with reference to the drawings.

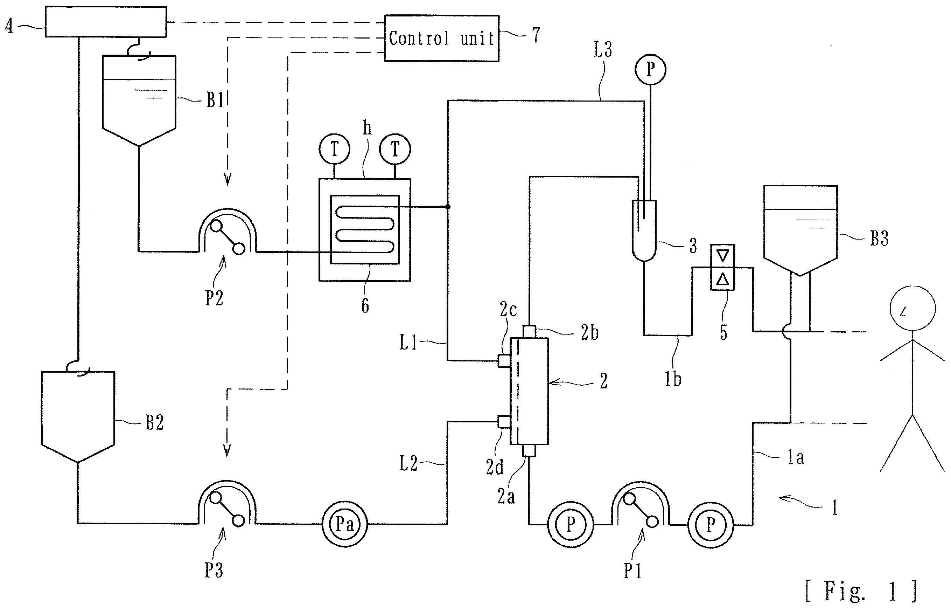

A blood purification apparatus according to a first embodiment is applied to a hemodialysis apparatus for purifying the blood of a patient while extracorporeally circulating the blood. As illustrated in Fig. 1, the blood purification apparatus includes a blood circuit 1 including an arterial blood circuit 1a and a venous blood circuit 1b, a dialyzer 2 (a blood purification device) interposed between the arterial blood circuit 1a and the venous blood circuit 1b and that purifies the blood flowing in the blood circuit 1, a blood pump P1 in the form of a peristaltic pump provided to the arterial blood circuit 1a, a dialysate introduction line L1 and a dialysate drain line L2, a dialysate pump P2 and a waste-liquid pump P3 provided to the dialysate introduction line L1 and the dialysate drain line L2, respectively, a substitution line L3, a dialysate storage bag B1 that stores dialysate to be introduced into the dialyzer 2, a waste-liquid storage bag B2 that stores waste liquid drained from the dialyzer 2, a priming-solution storage bag B3 that stores a priming solution, a weighing machine 4, and a control unit 7. Reference characters P and Pa given in the drawing each denote a pressure-detecting device (a pressure gauge), and a reference character T denotes a temperature-detecting device.

The arterial blood circuit 1a and the venous blood circuit 1b are provided at the distal ends thereof with connectors, respectively. An arterial puncture needle and a venous puncture needle (not illustrated) are connectable to the arterial blood circuit 1a and the venous blood circuit 1b with the connectors interposed therebetween, respectively. When the blood pump P1 is activated (to undergo normal rotation) with the arterial puncture needle at the distal end of the arterial blood circuit 1a and the venous puncture needle at the distal end of the venous blood circuit 1b being stuck in the patient, the blood of the patient flows through the arterial blood circuit 1a and reaches the dialyzer 2, where the blood is purified. Subsequently, the blood flows through the venous blood circuit 1b and returns into the body of the patient. Instead of sticking the arterial puncture needle and the venous puncture needle into a blood vessel of the patient, a double-lumen catheter may be inserted into the subclavian vein or the femoral vein of the patient or into a blood vessel in an arm of the patient, for example.

The venous blood circuit 1b is provided with an air-trap chamber 3 at a halfway position thereof. The blood that is extracorporeally circulated undergoes bubble removal in the air-trap chamber 3 and then returns into the patient. An air flow route extends from the top of the air-trap chamber 3. The air flow route is provided with a pressure-detecting device P at the distal end thereof. The pressure-detecting device P is capable of detecting the venous pressure. The venous blood circuit 1b is further provided with a clamping device 5 at a position of a distal portion thereof (at a position of the venous blood circuit 1b between the distal end and the air-trap chamber 3). The clamping device 5 is capable of opening and closing the flow route when opened and closed.

The substitution line L3 provides a flow route that allows a predetermined portion of the venous blood circuit 1b (in the present embodiment, the air-trap chamber 3) and the dialysate introduction line L1 to communicate with each other. The substitution line L3 allows dialysate serving as a substitution fluid to be supplied into the venous blood circuit 1b during blood purification treatment. In the present embodiment, the priming-solution storage bag B3 storing the priming solution is connected to the distal ends of the arterial blood circuit 1a and the venous blood circuit 1b. When the blood pump P1 is activated to rotate, the priming solution in the priming-solution storage bag B3 is supplied into the blood circuit 1, whereby priming can be performed.

The dialyzer 2 has, in a housing thereof, a blood inlet 2a (a blood introduction port), a blood outlet 2b (a blood delivery port), a dialysate inlet 2c (an inlet of the dialysate flow route, or a dialysate introduction port), and a dialysate outlet 2d (an outlet of the dialysate flow route, or a dialysate delivery port). The proximal end of the arterial blood circuit 1a is connected to the blood inlet 2a. The proximal end of the venous blood circuit 1b is connected to the blood outlet 2b. The dialysate inlet 2c and the dialysate outlet 2d are connected to the dialysate introduction line L1 and the dialysate drain line L2, respectively.

The dialyzer 2 houses a plurality of hollow fiber membranes (not illustrated), and such hollow fibers serve as blood purification membranes for purifying the blood. The blood purification membranes in the dialyzer 2 define blood flow routes (each extending between the blood inlet 2a and the blood outlet 2b) in which the blood of the patient flows and dialysate flow routes (each extending between the dialysate inlet 2c and the dialysate outlet 2d) in which the dialysate flows. The hollow fiber membranes serving as the blood purification membranes each have a number of very small holes (pores) extending therethrough from the outer peripheral surface to the inner peripheral surface. Hence, impurities and the like contained in the blood are allowed to penetrate through the membranes into the dialysate.

The dialysate introduction line L1 provides a flow route for introducing the dialysate into the dialyzer 2. One end of the dialysate introduction line L1 is connected to the dialysate inlet 2c of the dialyzer 2. The dialysate introduction line L1 is provided with the dialysate pump P2, which is a peristaltic pump that delivers liquid by squeezing a flexible tube forming the flow route provided by the dialysate introduction line L1. The other end of the dialysate introduction line L1 is connected to the dialysate storage bag B1 that stores a predetermined amount of dialysate. When the dialysate pump P2 is activated (to undergo normal rotation), the dialysate in the dialysate storage bag B1 can be introduced into the dialyzer 2.

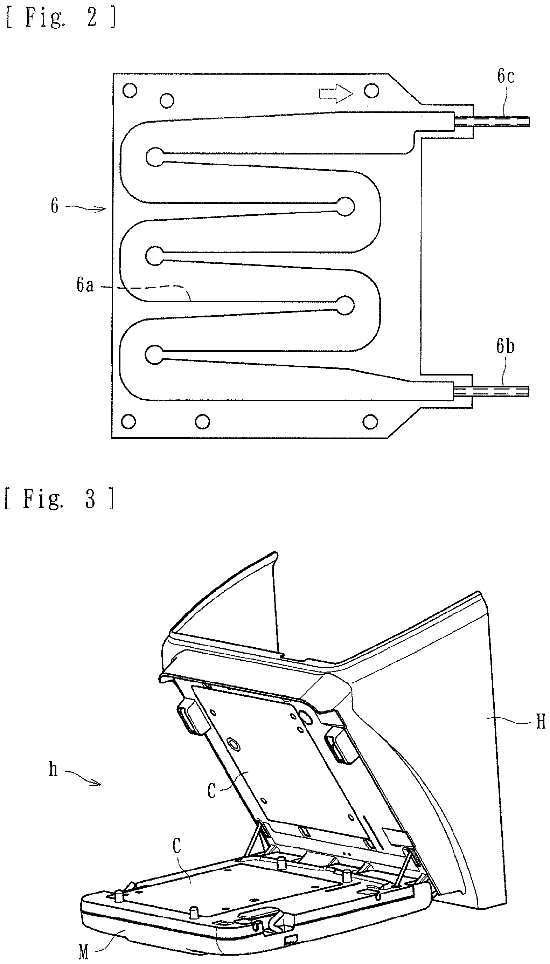

The dialysate introduction line L1 is further provided with a flexible heating bag 6 (a storage member) between the dialysate pump P2 and the connection to the substitution line L3. The heating bag 6 can store the dialysate flowing in the dialysate introduction line L1. The heating bag 6 is provided for heating the dialysate flowing in the dialysate introduction line L1 in the blood purification treatment. As illustrated in Fig. 2, the heating bag 6 is formed of two flexible sheets that are overlapped and welded to each other, whereby a flow route 6a is provided. The flow route 6a has connecting portions 6b and 6c at one end and the other end thereof, respectively. The connecting portions 6b and 6c are each connectable to the dialysate introduction line L1.

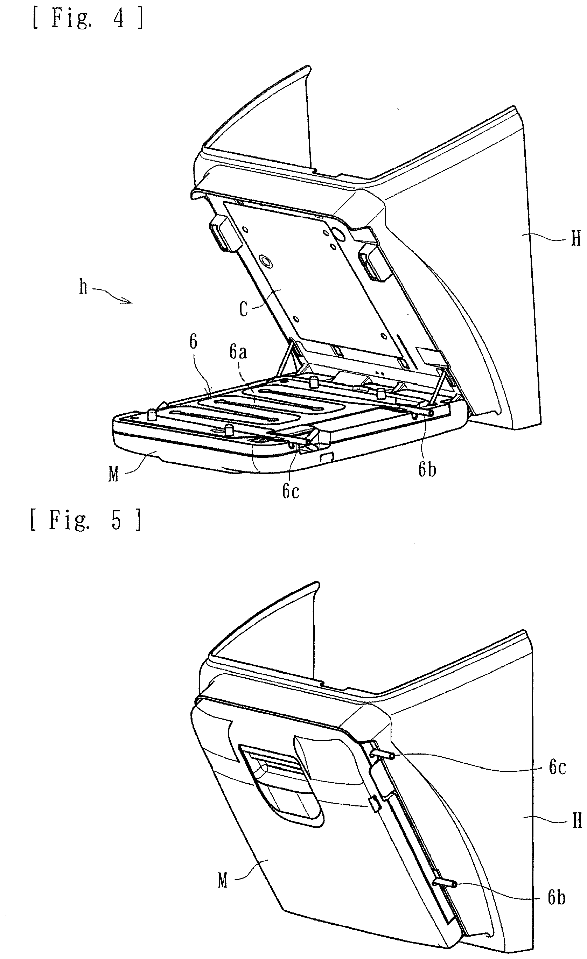

The heating bag 6 is attachable to a heating device h including a heater capable of heating the dialysate. As illustrated in Fig. 3, the heating device h is provided to a housing portion H that is attached to a dialysisapparatus body. The heating device h includes a lid portion M, and heating portions C. The heating portions C are provided to the housing portion H and the lid portion M, respectively. The heating portions C are each a metal plate member and are each capable of transmitting heat generated by a non-illustrated heat source (such as a heating wire). As illustrated in Fig. 4, the heating bag 6 is attached to the heating portion C provided to the lid portion M. Then, the lid portion M is closed as illustrated in Fig. 5. Thus, the heating bag 6 is held between the heating portions C provided to the housing portion H and the lid portion M, whereby the dialysate flowing in the flow route 6a can be heated with the heat generated by the heat sources.

As illustrated in Fig. 1, the dialysate drain line L2 provides a flow route for draining waste liquid from the dialyzer 2 and has one end thereof connected to the dialysate outlet 2d of the dialyzer 2. The dialysate drain line L2 is provided with the waste-liquid pump P3 in the form of a peristaltic pump that delivers liquid by squeezing a flexible tube forming the flow route provided by the dialysate drain line L2. The other end of the dialysate drain line L2 is connected to a waste-liquid storage bag B2 that can store a predetermined amount of waste liquid. When the waste-liquid pump P3 is activated (to undergo normal rotation), the dialysate (the waste liquid) drained from the dialyzer 2 can be introduced into the waste-liquid storage bag B2.

With the normal rotation of the dialysate pump P2, the dialysate in the dialysate storage bag B1 flows toward the dialyzer 2. With the normal rotation of the waste-liquid pump P3, the dialysate (the waste liquid) in the dialyzer 2 flows toward the waste-liquid storage bag B2. The dialysate storage bag B1 and the waste-liquid storage bag B2 are hung on respective hooks provided to the weighing machine 4. The weighing machine 4 is capable of measuring in real time the weight of the dialysate storage bag B1 and the waste-liquid storage bag B2 (the total weight of the dialysate storage bag B1 and the waste-liquid storage bag B2) (a single weighing machine 4 is shared by the dialysate storage bag B1 and the waste-liquid storage bag B2). The dialysate storage bag B1 and the waste-liquid storage bag B2 (and the priming-solution storage bag B3, as well) are each a flexible storage case. Before the blood purification treatment is started, the waste-liquid storage bag B2 is empty with no waste liquid stored thereinside.

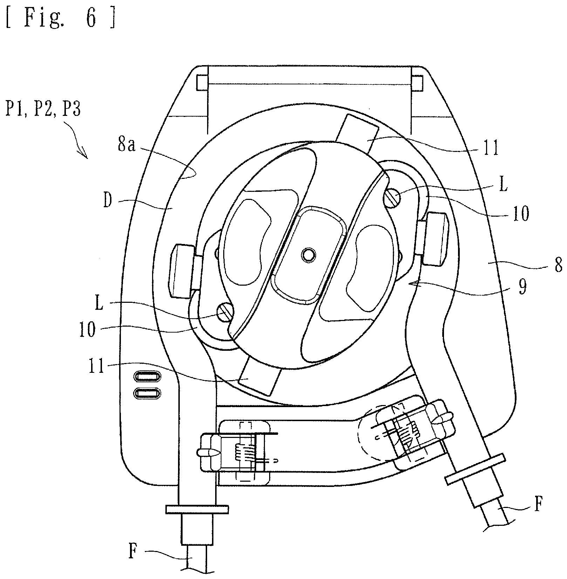

As illustrated in Fig. 6, the peristaltic pumps, such as the blood pump P1, the dialysate pump P2, and the waste-liquid pump P3, applied to the present embodiment each basically include a stator 8, a rotor 9 rotatable on the inner side of the stator 8, rollers 10 (squeezing units) provided to the rotor 9, and a pair of upper and lower guide pins 11. In the drawing, a cover provided over the stator 8 is not illustrated.

The stator 8 has a fitting recess 8a into which a squeezable tube D is fitted. The squeezable tube D can be fitted along the inner peripheral wall of the fitting recess 8a. The squeezable tube D is connected to a flexible tube F (the arterial blood circuit 1a for the blood pump P1, the dialysate introduction line L1 for the dialysate pump P2, or the flexible tube forming the flow route provided by the dialysate drain line L2 for the waste-liquid pump P3). The rotor 9, which is rotatably driven by a motor, is provided substantially at the center of the fitting recess 8a. The pair of rollers 10 and the guide pins 11 are provided on the side face (a surface facing the inner peripheral wall of the fitting recess 8a) of the rotor 9.

The rollers 10 are rotatable about respective rotating shafts L provided on the outer peripheral edge of the rotor 9. The rollers 10 compress the squeezable tube D, which is fitted in the fitting recess 8a, in the radial direction such that the flow route provided therein is closed. The rollers 10 also squeeze the squeezable tube D in the lengthwise direction (the direction in which the liquid flows) with the rotation of the rotor 9, whereby the liquid, such as the blood or the dialysate, is caused to flow. That is, when the rotor 9 is rotated with the squeezable tube D fitted in the fitting recess 8a, the squeezable tube D is compressed between the rollers 10 and the inner peripheral wall of the fitting recess 8a, whereby the flow route provided therein is closed (stopped). Furthermore, with the rotation of the rotor 9, the squeezable tube D can be squeezed in the direction of rotation of the rotor 9 (in the lengthwise direction). With such a squeezing motion, the liquid in the squeezable tube D is discharged in the direction of rotation of the rotor 9.

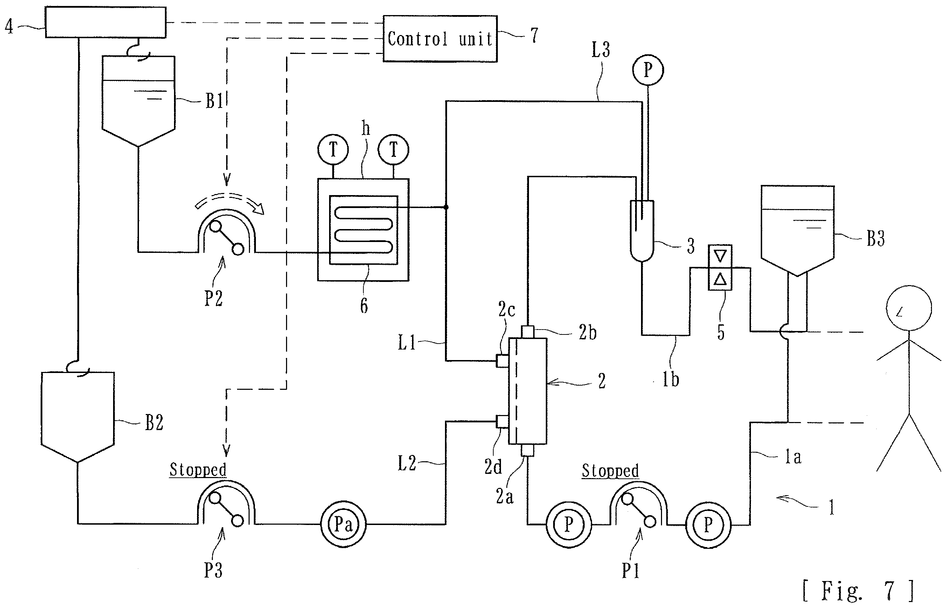

The control unit 7 illustrated in Fig. 1 is a microcomputer or the like provided in the blood purification apparatus and is capable of controlling the driving of the blood pump P1, the dialysate pump P2, and the waste-liquid pump P3 in accordance with the weight measured by the weighing machine 4 and pre-stored set values. The control unit 7 according to the present embodiment causes the dialysate pump P2 to rotate while keeping the waste-liquid pump P3 stopped, whereby the dialysate in the dialysate storage bag B1 is caused to flow without being stored in the waste-liquid storage bag B2. In accordance with a change in the value measured by the weighing machine 4 in the above process, the control unit 7 can calculate a correction value for the rotation speed of the dialysate pump P2 or for a value associated with the rotation speed, and can also execute a correcting process in which the rotation speed of the dialysate pump P2 is corrected. Specifically, as illustrated in Fig. 7, in the correcting process, the clamping device 5 is opened so that the flow route is opened, the dialysate pump P2 is activated (to undergo normal rotation), and the waste-liquid pump P3 is stopped. The blood pump P1 is preferably stopped.

In this state, the dialysate in the dialysate storage bag B1 flows into the dialysate introduction line L1 and then into the venous blood circuit 1b through the substitution line L3 but does not reach the waste-liquid storage bag B2. Hence, in the process of causing the dialysate in the dialysate storage bag B1 to flow into the venous blood circuit 1b, the rotation speed of the dialysate pump P2 can be corrected in accordance with the change in the value measured by the weighing machine 4. A correcting method will be described below. The liquid (the dialysate in the dialysate storage bag B1 and the priming solution or the like) that is caused to flow with the activation of the dialysate pump P2 is stored in the storage bag (in the present embodiment, the priming-solution storage bag B3) that is connected to the distal end of the venous blood circuit 1b.

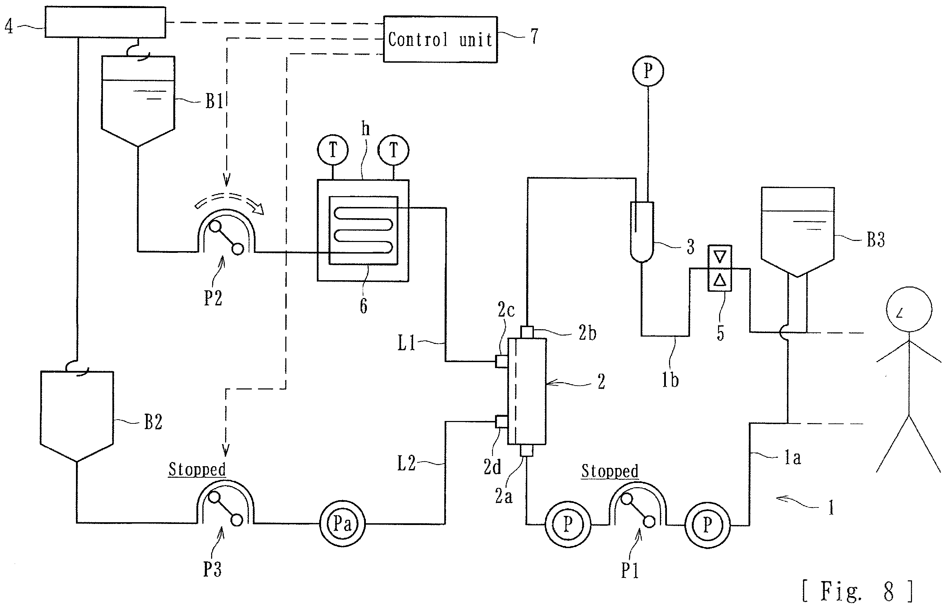

In a case illustrated in Fig. 8 where the substitution line L3 is not provided, the correcting process may be executed with the clamping device 5 being open so that the flow route is open, the dialysate pump P2 being activated (to undergo normal rotation), and the waste-liquid pump P3 being stopped as illustrated in the drawing. The blood pump P1 is preferably kept stopped. In this case, the dialysate in the dialysate storage bag B1 is allowed to flow into the venous blood circuit 1b through the dialyzer 2. That is, the dialysate in the dialysate storage bag B1 can be caused to flow into the venous blood circuit 1b without providing any additional dedicated flow route such as the substitution line L3.

According to the present embodiment, the control unit 7 activates the dialysate pump P2 to rotate, thereby causing the dialysate in the dialysate storage bag B1 to flow without being stored in the waste-liquid storage bag B2. Furthermore, in accordance with the change in the value measured by the weighing machine 4 in the above process, the control unit 7 can execute the correcting process in which the rotation speed of the dialysate pump P2 is corrected. Hence, while cost reduction is realized by measuring the weights of the dialysate storage bag B1 and the waste-liquid storage bag B2 with the single weighing machine 4, the rotation speed of the dialysate pump P2 can be corrected accurately, and the error in the flow rate of the dialysate to be introduced into the dialyzer 2 can be reduced.

In the correcting process, the control unit 7 according to the present embodiment activates the dialysate pump P2 to rotate while keeping the waste-liquid pump P3 stopped. Therefore, the dialysate flowing from the dialysate storage bag B1 in the correcting process can be assuredly prevented from being stored in the waste-liquid storage bag B2. Furthermore, the present embodiment employs the substitution line L3 that allows a predetermined portion of the venous blood circuit 1b and the dialysate introduction line L1 to communicate with each other, whereby, in the correcting process, the dialysate in the dialysate storage bag B1 can be caused to flow into the venous blood circuit 1b through the substitution line L3. That is, the dialysate can be caused to flow into the blood circuit 1 while detouring the dialyzer 2.

If the blood purification apparatus is configured such that the dialysate in the dialysate storage bag B1 is allowed to flow into the venous blood circuit 1b through the dialyzer 2, the dialysate in the dialysate storage bag B1 can be caused to flow into the venous blood circuit 1b without providing any additional dedicated flow route. Furthermore, in the correcting process, a storage bag (the priming-solution storage bag B3) that can store liquid is connected to the distal end of the venous blood circuit 1b. Therefore, the liquid flowing in the venous blood circuit 1b with the activation of the dialysate pump P2 can be prevented from spilling from the distal end of the venous blood circuit 1b.

If there is a treatment condition that ultrafiltration is not to be performed for the patient, the rotation speed of the waste-liquid pump P3 is controlled to be the same as the rotation speed of the dialysate pump P2. If ultrafiltration is to be performed, the rotation speed of the waste-liquid pump P3 is controlled to be higher than the rotation speed of the dialysate pump P2 by the flow rate for ultrafiltration. If there is any error, after a predetermined period of time, between the weight of the dialysate storage bag B1 and the waste-liquid storage bag B2 that is detected by the weighing machine 4 and the theoretical weight, the rotation speed of the waste-liquid pump P3 can be corrected automatically so that the error is eliminated.

That is, if the correction for the dialysate pump P2 according to the present invention and the correction for the waste-liquid pump P3 according to the prior art are combined together, the manufacturing cost can be reduced with a single weighing machine that weighs both the dialysate storage bag and the waste-liquid storage bag, the error in the flow rate of the dialysate to be introduced into the blood purification device can be reduced, and the error in the ultrafiltration that may occur in the treatment can be reduced.

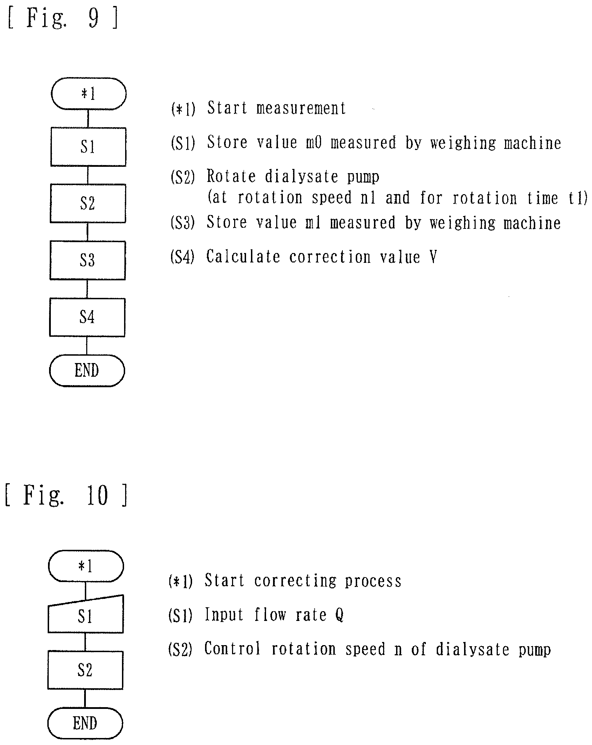

Now, a control operation in the correcting process performed by the control unit 7 according to the present embodiment will be described with reference to the flow charts illustrated in Figs. 9 and 10.

When a measurement process for the correction is started, as illustrated in Fig. 9, a value (m0) measured by the weighing machine 4 is stored in step S1. Subsequently, with the clamping device 5 being open as illustrated in Fig. 7 so that the flow route is open, the dialysate pump P2 is rotated (in the normal direction at a rotation speed n1 and for a rotation time t1) while the waste-liquid pump P3 is kept stopped (S2). Then, a value (m1) measured in this state by the weighing machine 4 is stored (S3).

Subsequently, a correction value V (the amount of discharge per revolution) is calculated (S4) in accordance with an arithmetic expression V = (m0-m1)/ρ/(n1·t1) (where p denotes the density of dialysate), using the measured value (m0) stored in step S1 and the measured value (m1) stored in step S3. The arithmetic expression used in step S4 provides a correction value for correcting the rotation speed of the dialysate pump P2 in accordance with the error between the value measured by the weighing machine 4 and the theoretical value.

This is the end of the measurement process for the correction. Subsequently, a correcting process is started. In the correcting process, as illustrated in Fig. 10, the rotation speed (n) of the dialysate pump P2 is corrected and controlled (S2) in accordance with a preset flow rate Q inputted by the operator (S1) and in accordance with an arithmetic expression: rotation speed (n) = preset flow rate (Q)/correction value (V). Thus, in the correcting process, the control unit 7 corrects and controls the rotation speed of the dialysate pump P2 in accordance with the error between the value measured by the weighing machine 4 and the theoretical value. Therefore, the rotation speed of the dialysate pump P2 can be corrected more accurately, and the flow-rate error attributed to the rotation of the dialysate pump P2 can be made smaller.

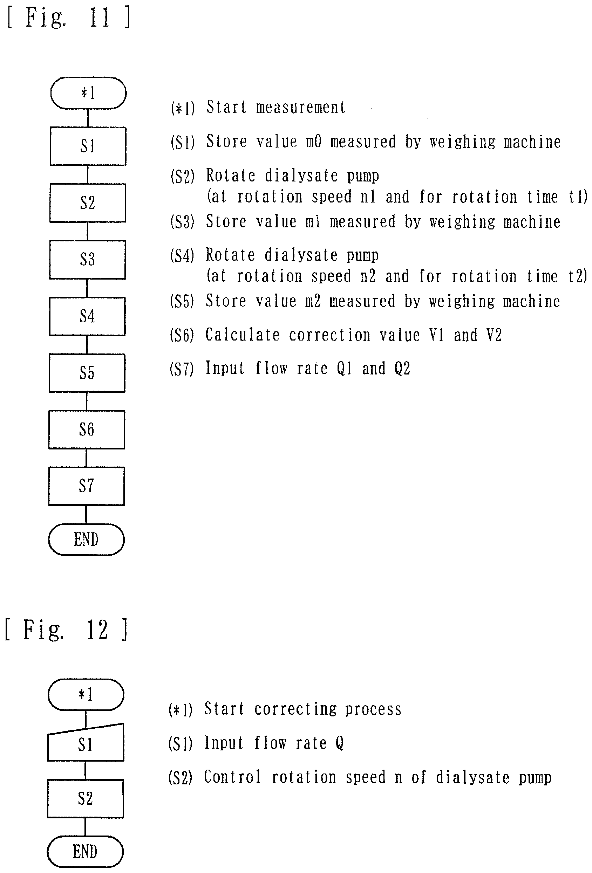

In a correcting process according to another embodiment, the control unit 7 performs a control operation based on the flow charts illustrated in Figs. 11 and 12.

When a measurement process for the correction is started, as illustrated in Fig. 11, a value (m0) measured by the weighing machine 4 is stored in step S1. Subsequently, with the clamping device 5 being open as illustrated in Fig. 7 so that the flow route is open, the dialysate pump P2 is rotated (in the normal direction at a rotation speed n1 and for a rotation time t1) while the waste-liquid pump P3 is kept stopped (S2). Then, a value (m1) measured in this state by the weighing machine 4 is stored (S3).

Furthermore, the dialysate pump P2 is rotated (in the normal direction) at a rotation speed n2 and for a rotation time t2 while the waste-liquid pump P3 is kept stopped (S4). Then, a value (m2) measured in this state by the weighing machine 4 is stored (S5). Subsequently, correction values V1 and V2 are calculated (S6) in accordance with arithmetic expressions V1 = (m0-m1) /ρ/ (n1·t1) and V2 = (m1-m2) /ρ/ (n2·t2) (where p denotes the density of dialysate), using the measured value (m0) stored in step S1, the measured value (m1) stored in step S3, and the measured value (m2) stored in step S5. The arithmetic expressions used in step S6 each provide a correction value for correcting the rotation speed of the dialysate pump P2 in accordance with the error among the values measured by the weighing machine 4 and the theoretical value. Subsequently, using the correction values V1 and V2, flow rates Q1 and Q2 are calculated (S7) in accordance with arithmetic expressions Q1 = n1·V1 and Q2 = n2·V2, respectively.

This is the end of the measurement process for the correction. Subsequently, a correcting process is started. In the correcting process, as illustrated in Fig. 12, the rotation speed (n) of the dialysate pump P2 is corrected and controlled in accordance with a preset flow rate Q inputted by the operator (S1) and in accordance with an arithmetic expression: rotation speed (n) = (n1/Q1)·Q if a relationship 0 ≤ Q < Q1 holds, or an arithmetic expression: rotation speed (n) = ((n2-n1)/(Q2-Q1))·Q+(n1-((n2-n1)/(Q2-Q1))·Q1) if a relationship Q ≥ Q2 holds. Thus, in the correcting process, the control unit 7 can activate the dialysate pump P2 at a plurality of rotation speeds and can set correction values corresponding to the respective rotation speeds of the dialysate pump P2 in accordance with the changes in the values measured by the weighing machine 4 that are obtained at the respective rotation speeds. Therefore, the flow-rate error attributed to the rotation of the dialysate pump P2 can be made much smaller.

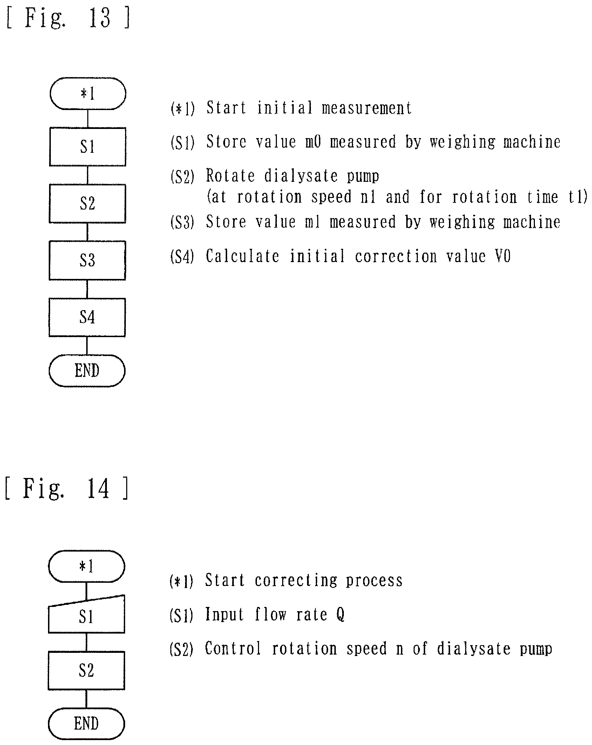

In a correcting process according to yet another embodiment, the control unit 7 performs a control operation based on the flow charts illustrated in Figs. 13 and 14.

When a measurement process for the correction is started, as illustrated in Fig. 13, a value (m0) measured by the weighing machine 4 is stored in step S1. Subsequently, with the clamping device 5 being open as illustrated in Fig. 7 so that the flow route is open, the dialysate pump P2 is rotated (in the normal direction at a rotation speed n1 and for a rotation time t1) while the waste-liquid pump P3 is kept stopped (S2). Then, a value (ml) measured in this state by the weighing machine 4 is stored (S3).

Subsequently, a correction value V0 (the initial value for the amount of discharge per revolution) is calculated (S4) in accordance with an arithmetic expression V0 = (m0-m1)/ρ/(n1·t1) (where p denotes the density of dialysate), using the measured value (m0) stored in step S1 and the measured value (ml) stored in step S3. The arithmetic expression used in step S4 provides a correction value for correcting the rotation speed of the dialysate pump P2 in accordance with the error between the value measured by the weighing machine 4 and the theoretical value.

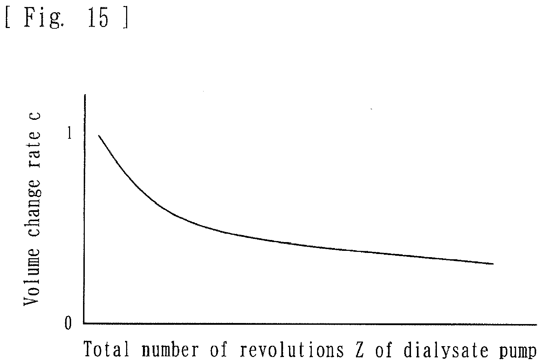

This is the end of the measurement process for the correction. Subsequently, a correcting process is started. Prior to the correcting process, characteristic data on the rate of change in the flow rate (the volume change rate) with respect to a total number of revolutions Z of the dialysate pump P2 illustrated in Fig. 15 is stored. When the correcting process is started, as illustrated in Fig. 14, the control unit 7 corrects and controls the rotation speed (n) of the dialysate pump P2 (S2) in accordance with a preset flow rate Q inputted by the operator (S1) and the pre-stored characteristic data and in accordance with an arithmetic expression: rotation speed (n) = preset flow rate (Q)/(correction value (V0)·volume change rate (c)).

Thus, the control unit 7 can store in advance the characteristic data on the rate of change in the flow rate (the volume change rate c) with respect to the total number of revolutions Z of the dialysate pump P2. Furthermore, in the correcting process, the control unit 7 can set a correction value corresponding to the rotation speed of the dialysate pump P2 in accordance with the characteristic data. Therefore, even if any flow-rate error occurs because of the aging of the dialysate pump P2, the control unit 7 can set a correction value corresponding to the rotation speed of the dialysate pump P2 more smoothly without activating the dialysate pump P2 at a plurality of rotation speeds.

Now, a blood purification apparatus according to a second embodiment of the present invention will be described.

As with the case of the first embodiment, the blood purification apparatus according to the second embodiment is applied to a hemodialysis apparatus for purifying the blood of a patient while extracorporeally circulating the blood. As illustrated in Fig. 1, the blood purification apparatus includes a blood circuit 1 including an arterial blood circuit 1a and a venous blood circuit 1b, a dialyzer 2 (a blood purification device) interposed between the arterial blood circuit 1a and the venous blood circuit 1b and that purifies the blood flowing in the blood circuit 1, a blood pump P1 in the form of a peristaltic pump provided to the arterial blood circuit 1a, a dialysate introduction line L1 and a dialysate drain line L2, a dialysate pump P2 and a waste-liquid pump P3 provided to the dialysate introduction line L1 and the dialysate drain line L2, respectively, a substitution line L3, a dialysate storage bag B1 that stores dialysate to be introduced into the dialyzer 2, a waste-liquid storage bag B2 that stores waste liquid drained from the dialyzer 2, a priming-solution storage bag B3 that stores a priming solution, a weighing machine 4, and a control unit 7. Elements that are the same as those described in the first embodiment are denoted by corresponding ones of the reference numerals, and detailed description of those elements is omitted.

The control unit 7 according to the present embodiment causes the dialysate pump P2 to rotate while keeping the waste-liquid pump P3 stopped, whereby the dialysate in the dialysate storage bag B1 is caused to flow without being stored in the waste-liquid storage bag B2. In accordance with a change in the value measured by the weighing machine 4 in the above process, the control unit 7 can execute a correcting process in which the rotation speed of the dialysate pump P2 is corrected. In the correcting process, the dialysate in the dialysate storage bag B1 can be stored in the heating bag 6 (a storage member) connected to the dialysate introduction line L1.

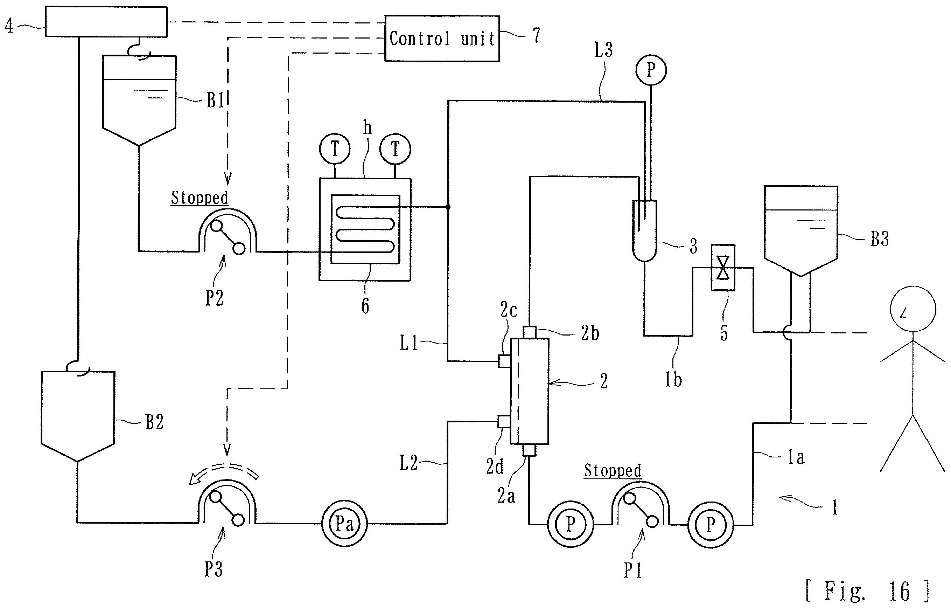

Specifically, as illustrated in Fig. 16, in the correcting process, before the dialysate pump P2 is rotated as described above, the waste-liquid pump P3 is activated (to undergo normal rotation) while the dialysate pump P2 is kept stopped (in this step, the clamping device 5 is closed). Hence, the liquid in the heating bag 6 (the storage member) is discharged, and a capacity for storing the liquid that is to flow when the dialysate pump P2 is activated is secured. After the waste-liquid pump P3 is activated (to undergo normal rotation), the waste-liquid pump P3 is preferably stopped on condition that the value measured by the pressure-detecting device (a pressure gauge) attached to the dialysate drain line L2 reaches a predetermined value. Such a method can prevent generation of bubbles that may occur if the pressure inside the dialysate introduction line L1 becomes negative or liquid leakage or the like that may occur if the pressure becomes too high.

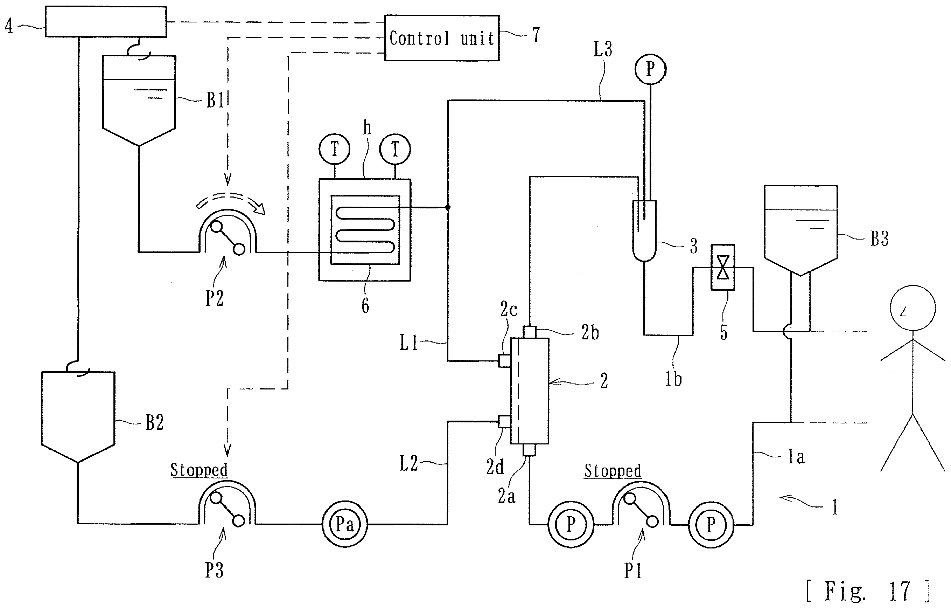

Subsequently, as illustrated in Fig. 17, while the clamping device 5 is kept closed so that the flow route is closed, the dialysate pump P2 is activated (to undergo normal rotation) but the waste-liquid pump P3 is stopped. In this state, the blood pump P1 is preferably kept stopped. Thus, the dialysate in the dialysate storage bag B1 is stored in the heating bag 6 but does not reach the waste-liquid storage bag B2. When the dialysate is stored in the heating bag 6, which is made of a flexible material, the heating bag 6 inflates in accordance with the amount of dialysate that flows thereinto. Hence, in the process of causing the dialysate in the dialysate storage bag B1 to be stored in the heating bag 6, the rotation speed of the dialysate pump P2 can be corrected in accordance with the change in the value measured by the weighing machine 4. The correction is performed by the same method as in the first embodiment (including the another and the yet another embodiments).

According to the present embodiment, the control unit 7 activates the dialysate pump P2 to rotate, thereby causing the dialysate in the dialysate storage bag B1 to flow without being stored in the waste-liquid storage bag B2. In accordance with the change in the value measured by the weighing machine 4 in the above process, the control unit 7 can execute the correcting process in which the rotation speed of the dialysate pump P2 is corrected. Hence, while cost reduction is realized by measuring the weights of the dialysate storage bag B1 and the waste-liquid storage bag B2 with the single weighing machine 4, the rotation speeds of the dialysate pump P2 and the waste-liquid pump P3 can be corrected accurately, and the error in the flow rate of the dialysate to be introduced into the dialyzer 2 can be reduced.

In the correcting process, the control unit 7 according to the present embodiment activates the dialysate pump P2 to rotate while keeping the waste-liquid pump P3 stopped. Therefore, in the correcting process, the dialysate flowing from the dialysate storage bag B1 can be assuredly prevented from being stored in the waste-liquid storage bag B2. Furthermore, the dialysate introduction line L1 is connected to the heating bag 6 serving as a flexible storage member capable of storing the dialysate flowing in the dialysate introduction line L1. Moreover, the dialysate in the dialysate storage bag B1 can be stored in the heating bag 6 in the correcting process. Therefore, the liquid that is caused to flow with the activation of the dialysate pump P2 can be prevented from flowing into the blood circuit 1.

In particular, since the heating bag 6 serving as a storage member is flexible, the heating bag 6 is inflatable in accordance with the flow rate of the liquid that is caused to flow with the activation of the dialysate pump P2. Hence, the occurrence of resistance (flow resistance) at the flowing of the liquid can be suppressed, and the rotation speed of the dialysate pump P2 can be corrected more accurately. Furthermore, since the storage member is provided as the heating bag 6 for heating the dialysate flowing in the dialysate introduction line L1 during the blood purification treatment, the liquid that is caused to flow with the activation of the dialysate pump P2 can be stored by utilizing the heating bag 6, which is necessary for the blood purification treatment. In addition, in the correcting process, the control unit 7 can cause the liquid in the heating bag 6 to be discharged before activating the dialysate pump P2, thereby securing a capacity for storing the liquid that is to flow when the dialysate pump P2 is activated (to undergo normal rotation). Therefore, the liquid that is caused to flow with the activation of the dialysate pump P2 can be more assuredly stored in the heating bag 6.

After the correcting process according to each of the first and second embodiments, the dialysate pump P2 and the waste-liquid pump P3 are activated simultaneously, and the rotation speed of the waste-liquid pump P3 is corrected in accordance with the change in the value measured by the weighing machine 4. That is, since the dialysate pump P2 is regarded as having no flow-rate error attributed to the activation thereof (or has a reduced flow-rate error), the error detected in accordance with the change in the value measured by the weighing machine 4 can be regarded as an error in the waste-liquid pump P3, and the waste-liquid pump P3 can be corrected accordingly.

The correcting process according to each of the first and second embodiments is performed prior to the blood purification treatment (after the completion of priming), and the blood purification treatment can be started immediately thereafter. Alternatively, the correcting process may be performed during the blood purification treatment (during a period over which the blood of the patient is extracorporeally circulated through the blood circuit 1 with the puncture needles at the distal ends of the arterial blood circuit 1a and the venous blood circuit 1b being stuck in the patient). In such a case, even if any change occurs in the characteristic of the dialysate pump P2 during the blood purification treatment, the rotation speed of the dialysate pump P2 can be corrected.

While several embodiments have been described above, the present invention, defined by the appended claims, is not limited thereto. For example, the waste-liquid pump P3 may be activated in the correcting process, as long as the rotation speed of the dialysate pump P2 is correctable in accordance with the change in the value measured by the weighing machine 4 in the process of activating the dialysate pump P2 such that the dialysate in the dialysate storage bag B1 is caused to flow without being stored in the waste-liquid storage bag B2.

Alternatively, after a correction value for the rotation speed (including the value associated with the rotation speed) of the dialysate pump P2 is calculated by the control unit 7, the rotation speed of the dialysate pump P2 may be corrected manually in accordance with the correction value. The correction value (including the rotation speed corrected in accordance with the correction value) is preferably notified by a notification device. Examples of the notification device include a display device that displays the correction value, an audio output device that outputs the correction value in the form of audio, a lamp that is turned on or off if the actual rotation speed of the dialysate pump P2 matches or does not match the corrected rotation speed. Such an embodiment produces an advantageous effect of being capable of helping (assisting) accurate manual correction of the dialysate pump P2.

In the first embodiment, an electromagnetic valve that is opened in the correcting process may be provided to the substitution line L3. In the second embodiment, instead of the heating bag 6, another flexible storage member (that is, a storage case that is inflatable in accordance with the flow rate of the dialysate) may be provided to the dialysate introduction line L1. The applicable blood purification treatment is not limited to dialysis treatment and may be another treatment for purifying the blood of a patient while extracorporeally circulating the blood.

### Industrial Applicability

The blood purification apparatus may have other additional functions or the like, as long as the blood purification apparatus includes a control unit that is capable of calculating a correction value for the rotation speed of a dialysate pump or for a value associated with the rotation speed in accordance with the change in the value measured by a weighing machine in a process of activating a dialysate pump such that dialysate in a dialysate storage bag is caused to flow without being stored in a waste-liquid storage bag.

### Reference Signs List

- 1: blood circuit
- 1a: arterial blood circuit
- 1b: venous blood circuit
- 2: dialyzer (blood purification device)
- 3: air-trap chamber
- 4: weighing machine
- 5: clamping device
- 6: heating bag (storage member)
- 7: control unit
- h: heating device
- L1: dialysate introduction line
- L2: dialysate drain line
- L3: substitution line
- P1: blood pump
- P2: dialysate pump
- P3: waste-liquid pump

## Claims

1. A blood purification apparatus that includes
a blood circuit (1) including an arterial blood circuit (1a) and a venous blood circuit (2a) and being capable of extracorporeally circulating blood of a patient from a distal end of the arterial blood circuit (1a) to a distal end of the venous blood circuit (2a);
a blood purification device (2) interposed between the arterial blood circuit (1a) and the venous blood circuit (2a) of the blood circuit (1) and being capable of purifying the blood flowing in the blood circuit (1);
a dialysate introduction line (L1) for introducing dialysate into the blood purification device (2),
and a dialysate drain line (L2) for draining waste liquid from the blood purification device (2);
a dialysate pump (P2) in a form of a peristaltic pump provided to the dialysate introduction line (L1), the dialysate pump (P2) being capable of causing the dialysate in the dialysate introduction line (L1) to flow when the dialysate pump (P2) is activated to rotate;
a dialysate storage bag (B1) connected to a distal end of the dialysate introduction line (L1) and that stores the dialysate to be introduced into the blood purification device (2);
a waste-liquid pump (P3) in a form of a peristaltic pump provided to the dialysate drain line (L2), the waste-liquid pump (P3) being capable of causing the waste liquid in the dialysate drain line (L2) to flow when the waste-liquid pump (P3) is activated to rotate;
a waste-liquid storage bag (B2) connected to a distal end of the dialysate drain line (L2) and that stores the waste liquid drained from the blood purification device (2);
a single weighing machine (4) capable of measuring a total weight of the dialysate storage bag (B1) and the waste-liquid storage bag (B2); and
a control unit (7) configured to control the dialysate pump (P2) and the waste-liquid pump (P3) in accordance with a value measured by the weighing machine (4),
wherein the control unit (7) is configured to calculate a correction value for a rotation speed of the dialysate pump (P2) or for a value associated with the rotation speed in accordance with a change in the value measured by the weighing machine (4) in a process of activating the dialysate pump (P2) such that the dialysate in the dialysate storage bag (B1) is caused to flow without being stored in the waste-liquid storage bag (B2),
**characterized in that**
the control unit (7) is configured to execute a correcting process in which the rotation speed of the dialysate pump (P2) or the value associated with the rotation speed is corrected in accordance with the calculated correction value and in which the control unit (7) activates the dialysate pump (P2) to rotate while keeping the waste-liquid pump (P3) stopped.

2. The blood purification apparatus according to Claim 1, **characterized in that** the dialysate in the dialysate storage bag (B1) is allowed to flow into the venous blood circuit (2a) through the blood purification device (2).

3. The blood purification apparatus according to Claim 1, **characterized by** further including a substitution line (L3) that allows a predetermined portion of the venous blood circuit (2a) and the dialysate introduction line (L1) to communicate with each other, the apparatus also being **characterized in that**, in the correcting process, the dialysate in the dialysate storage bag (B1) is allowed to flow into the venous blood circuit (2a) through the substitution line (L3).

4. The blood purification apparatus according to Claim 2 or 3, **characterized in that**, in the correcting process, a storage bag (B3) capable of storing liquid is connected to the distal end of the venous blood circuit (2a).

5. The blood purification apparatus according to Claim 1, **characterized in that** the dialysate introduction line (L1) is provided with a flexible storage member that is capable of storing the dialysate flowing in the dialysate introduction line (L1); and **in that**, in the correcting process, the dialysate in the dialysate storage bag (B1) is allowed to be stored in the storage member.

6. The blood purification apparatus according to Claim 5, **characterized in that**, in the correcting process, the control unit (7) is configured to cause the liquid in the storage member to be discharged before activating the dialysate pump (P2) to rotate, and is configured to secure a capacity for storing the liquid that is to flow with an activation of the dialysate pump (P2).

7. The blood purification apparatus according to Claim 5 or 6, **characterized in that** the storage member is a heating bag (6) for heating the dialysate flowing in the dialysate introduction line (L1) in blood purification treatment.

8. The blood purification apparatus according to any of Claims 1 to 7, **characterized in that** the correcting process is performed before blood purification treatment.

9. The blood purification apparatus according to any of Claims 1 to 7, **characterized in that** the correcting process is performed during blood purification treatment.

10. The blood purification apparatus according to any of Claims 1 to 9, **characterized in that**, in the correcting process, the control unit corrects and controls the rotation speed of the dialysate pump (P2) in accordance with an error between a value measured by the weighing machine (4) and a theoretical value.

11. The blood purification apparatus according to any of Claims 1 to 10, **characterized in that**, in the correcting process, the control unit is configured to activate the dialysate pump (P2) at a plurality of rotation speeds and to set correction values corresponding to the respective rotation speeds of the dialysate pump (P2) in accordance with changes in the values measured by the weighing machine (4) that are obtained at the respective rotation speeds.

12. The blood purification apparatus according to any of Claims 1 to 10, **characterized in that** characteristic data on a rate of change in a flow rate with respect to a total number of revolutions of the dialysate pump (P2) is stored in advance; and **in that**, in the correcting process, the control unit is configured to set a correction value corresponding to the rotation speed of the dialysate pump (P2) in accordance with the characteristic data.

## Patentansprüche

1. Blutreinigungsapparat, enthaltend
einen Blutkreislauf (1), der einen arteriellen Blutkreislauf (1a) und einen venösen Blutkreislauf (2a) aufweist und in der Lage ist, Blut eines Patienten extrakorporal von einem distalen Ende des arteriellen Blutkreislaufs (1a) zu einem distalen Ende des venösen Blutkreislaufs (2a) zu zirkulieren;
eine Blutreinigungsvorrichtung (2), die zwischen dem arteriellen Blutkreislauf (1a) und dem venösen Blutkreislauf (2a) des Blutkreislaufs (1) angeordnet und in der Lage ist, das in dem Blutkreislauf (1) fließende Blut zu reinigen;
eine Dialysateinführungsleitung (L1) zum Einführen von Dialysat in die Blutreinigungsvorrichtung (2), und eine Dialysatabflussleitung (L2) zum Ablassen von Abfallflüssigkeit aus der Blutreinigungsvorrichtung (2);
eine Dialysatpumpe (P2) in Form einer Peristaltikpumpe, die an der Dialysateinführungsleitung (L1) vorgesehen ist, wobei die Dialysatpumpe (P2) in der Lage ist, das Dialysat in der Dialysateinführungsleitung (L1) zum Fließen zu bringen, wenn die Dialysatpumpe (P2) aktiviert wird, um sich zu drehen;
einen Dialysatvorratsbeutel (B1), der mit einem distalen Ende der Dialysateinführungsleitung (L1) verbunden ist und der das in die Blutreinigungsvorrichtung (2) einzuführende Dialysat speichert; eine Abfallflüssigkeitspumpe (P3) in Form einer Peristaltikpumpe, die an der Dialysatabflussleitung (L2) vorgesehen ist, wobei die Abfallflüssigkeitspumpe (P3) in der Lage ist, die Abfallflüssigkeit in der Dialysatabflussleitung (L2) zum Fließen zu bringen, wenn die Abfallflüssigkeitspumpe (P3) aktiviert wird, um sich zu drehen;
einen Abfallflüssigkeitsspeicherbeutel (B2), der mit einem distalen Ende der Dialysatabflussleitung (L2) verbunden ist und der die aus der Blutreinigungsvorrichtung (2) abgeleitete Abfallflüssigkeit speichert;
eine Einzelwaage (4), die in der Lage ist, das Gesamtgewicht des Dialysatvorratsbeutels (B1) und des Abfallflüssigkeitsspeicherbeutels (B2) zu messen; und
eine Steuereinheit (7), die ausgebildet ist, um die Dialysatpumpe (P2) und die Abfallflüssigkeitspumpe (P3) in Abhängigkeit von einem von der Wiegemaschine (4) gemessenen Wert zu steuern,
wobei die Steuereinheit (7) ausgebildet ist, um einen Korrekturwert für eine Rotationsgeschwindigkeit der Dialysatpumpe (P2) oder für einen mit der Rotationsgeschwindigkeit in Beziehung stehenden Wert in Abhängigkeit von einer Änderung des von der Wiegemaschine (4) gemessenen Wertes in einem Prozess der Aktivierung der Dialysatpumpe (P2) zu berechnen, so dass das Dialysat in dem Dialysatvorratsbeutel (B1) veranlasst wird zu fließen, ohne in dem Abfallflüssigkeitsspeicherbeutel (B2) gespeichert zu werden,
**dadurch gekennzeichnet, dass**
die Steuereinheit (7) ausgebildet ist, um einen Korrekturvorgang auszuführen, bei dem die Drehgeschwindigkeit der Dialysatpumpe (P2) oder der mit der Drehgeschwindigkeit verbundene Wert gemäß dem berechneten Korrekturwert korrigiert wird und bei dem die Steuereinheit (7) die Dialysatpumpe (P2) zum Drehen aktiviert, während die Abfallflüssigkeitspumpe (P3) angehalten bleibt.

2. Blutreinigungsapparat nach Anspruch 1, **dadurch gekennzeichnet, dass** das Dialysat im Dialysatvorratsbeutel (B1) durch die Blutreinigungsvorrichtung (2) in den venösen Blutkreislauf (2a) fließen kann.

3. Blutreinigungsapparat nach Anspruch 1, **dadurch gekennzeichnet, dass** er ferner eine Substitutionsleitung (L3) aufweist, die es ermöglicht, dass ein vorbestimmter Abschnitt des venösen Blutkreislaufs (2a) und die Dialysateinleitungsleitung (L1) miteinander in Verbindung stehen, wobei der Apparatauch **dadurch gekennzeichnet ist, dass** man bei dem Korrekturvorgang das Dialysat in dem Dialysatvorratsbeutel (B1) durch die Substitutionsleitung (L3) in den venösen Blutkreislauf (2a) fließen lässt.

4. Blutreinigungsapparat nach Anspruch 2 oder 3, **dadurch gekennzeichnet, dass** beim Korrekturvorgang ein Speicherbeutel (B3), der Flüssigkeit speichern kann, an das distale Ende des venösen Blutkreislaufs (2a) angeschlossen wird.

5. Blutreinigungsapparat nach Anspruch 1, **dadurch gekennzeichnet, dass** die Dialysat-Zuführungsleitung (L1) mit einem flexiblen Speicherelement versehen ist, das in der Lage ist, das in der Dialysat-Zuführungsleitung (L1) fließende Dialysat zu speichern; und dass im Korrekturvorgang das Dialysat im Dialysat-Speicherbeutel (B1) in dem Speicherelement gespeichert werden kann.

6. Blutreinigungsapparat nach Anspruch 5, **dadurch gekennzeichnet, dass** die Steuerung (7) ausgebildet ist, um bei dem Korrekturvorgang vor der Aktivierung der Dialysatpumpe (P2) zum Drehen die Flüssigkeit in dem Speicherelement abfließen zu lassen, und ausgebildet ist, um eine Kapazität zum Speichern der Flüssigkeit, die bei einer Aktivierung der Dialysatpumpe (P2) fließen soll, zu sichern.

7. Blutreinigungsapparat nach Anspruch 5 oder 6, **dadurch gekennzeichnet, dass** das Speicherelement ein Heizbeutel (6) zur Erwärmung des bei der Blutreinigungsbehandlung in der Dialysateinleitungsleitung (L1) fließenden Dialysats ist.

8. Blutreinigungsapparat nach einem der Ansprüche 1 bis 7, **dadurch gekennzeichnet, dass** der Korrekturvorgang vor der Blutreinigungsbehandlung durchgeführt wird.

9. Blutreinigungsapparat nach einem der Ansprüche 1 bis 7, **dadurch gekennzeichnet, dass** der Korrekturvorgang während der Blutreinigungsbehandlung durchgeführt wird.

10. Blutreinigungsapparat nach einem der Ansprüche 1 bis 9, **dadurch gekennzeichnet, dass** die Steuerung bei dem Korrekturvorgang die Drehzahl der Dialysatpumpe (P2) in Abhängigkeit von einem Fehler zwischen einem von der Waage (4) gemessenen Wert und einem theoretischen Wert korrigiert und steuert.

11. Blutreinigungsapparat nach einem der Ansprüche 1 bis 10, **dadurch gekennzeichnet, dass** die Steuerung bei dem Korrekturvorgang ausgebildet ist, die Dialysatpumpe (P2) mit einer Vielzahl von Drehzahlen zu aktivieren und Korrekturwerte entsprechend den jeweiligen Drehzahlen der Dialysatpumpe (P2) in Abhängigkeit von Änderungen der von der Waage (4) gemessenen Werte, die bei den jeweiligen Drehzahlen erlangt werden, einzustellen.

12. Blutreinigungsapparat nach einem der Ansprüche 1 bis 10, **dadurch gekennzeichnet, dass** charakteristische Daten über eine Änderungsrate einer Flussrate in Bezug auf eine Gesamtzahl von Umdrehungen der Dialysatpumpe (P2) im Voraus gespeichert werden und dass die Steuereinheit ausgebildet ist, um bei dem Korrekturvorgang einen Korrekturwert entsprechend der Drehgeschwindigkeit der Dialysatpumpe (P2) in Übereinstimmung mit den charakteristischen Daten einzustellen.

## Revendications

1. Appareil de purification de sang comprenant :
un circuit sanguin (1) comportant un circuit sanguin artériel (1a) et un circuit sanguin veineux (2a) et apte à circuler, de manière extracorporelle, le sang d'un patient depuis une extrémité distale du circuit sanguin artériel (1a) à une extrémité distale du circuit sanguin veineux (2a) ;
un dispositif (2) de purification de sang disposé entre le circuit sanguin artériel (1a) et le circuit sanguin veineux (2a) du circuit sanguin (1) et apte à purifier le sang circulant dans le circuit sanguin (1) ;
une ligne (L1) d'introduction de dialysat pour introduire du dialysat dans ledit dispositif (2) de purification de sang, et une ligne (L2) de déchargement de dialysat pour décharger le liquide usagé depuis ledit dispositif (2) de purification de sang ;
une pompe (P2) à dialysat en forme de pompe péristaltique dont ladite ligne (L1) d'introduction de dialysat est dotée, ladite pompe (P2) à dialysat étant apte à faire circuler le dialysat dans ladite ligne (L1) d'introduction de dialysat lorsque la pompe (P2) à dialysat est activée à tourner ;
une poche (B1) de stockage de dialysat qui est connectée à une extrémité distale de ladite ligne (L1) d'introduction de dialysat et qui est destinée à stocker le dialysat à introduire dans ledit dispositif (2) de purification de sang ;
une pompe (P3) à liquide usagé en forme de pompe péristaltique dont ladite ligne (L2) de déchargement de dialysat est dotée, ladite pompe (P3) à liquide usagé étant apte à faire circuler le liquide usagé dans ladite ligne (L2) de déchargement de dialysat lorsque la pompe (P3) à liquide usagé est activée à tourner ;
une poche (B2) de stockage de liquide usagé qui est connectée à une extrémité distale de ladite ligne (L2) de déchargement de dialysat et qui est destinée à stocker le liquide usagé déchargé depuis ledit dispositif (2) de purification de sang ;
une machine unique de pesage (4) apte à mesurer un poids total de ladite poche (B1) de stockage de dialysat et de ladite poche (B2) de stockage de liquide usagé ; et
une unité de commande (7) configurée pour commander la pompe (P2) à dialysat et la pompe (P3) à liquide usagé en fonction d'une valeur mesurée par ladite machine de pesage (4),
ladite unité de commande (7) étant configurée pour calculer une valeur de correction pour une vitesse de rotation de la pompe (P2) à dialysat ou pour une valeur associée à la vitesse de rotation en fonction d'une variation de la valeur mesurée par ladite machine de pesage (4) dans un processus pour activer la pompe (P2) à dialysat de façon que le dialysat dans ladite poche (B1) de stockage de dialysat est fait circuler sans être stocké dans ladite poche (B2) de stockage de liquide usagé,
**caractérisé en ce que**
ladite unité de commande (7) est configurée pour effectuer un processus de correction dans lequel la vitesse de rotation de la pompe (P2) à dialysat ou la valeur associée à la vitesse de rotation est corrigée en fonction de la valeur de correction calculée et dans lequel ladite unité de commande (7) est destinée à activer la pompe (P2) à dialysat à tourner tout en maintenant arrêtée ladite pompe (P3) à liquide usagé.

2. Appareil de purification de sang selon la revendication 1, **caractérisé en ce que** le dialysat dans ladite poche (B1) de stockage de dialysat est permis à entrer dans le circuit sanguin veineux (2a) en s'écoulant à travers ledit dispositif (2) de purification de sang.

3. Appareil de purification de sang selon la revendication 1, **caractérisé en ce qu'**il comprend en outre une ligne de substitution (L3) qui permet à une partie prédéterminée dudit circuit sanguin artériel (2a) et à ladite ligne (L1) d'introduction de dialysat de communiquer entre eux, ledit appareil étant également **caractérisé en ce que**, dans le processus de correction, le dialysat dans ladite poche (B1) de stockage de dialysat est permis à entrer dans le circuit sanguin veineux (2a) en s'écoulant à travers ladite ligne de substitution (L3).

4. Appareil de purification de sang selon la revendication 2 ou 3, **caractérisé en ce que**, dans le processus de correction, une poche (B3) de stockage apte à stocker du liquide est connectée à l'extrémité distale du circuit sanguin veineux (2a).

5. Appareil de purification de sang selon la revendication 1, **caractérisé en ce que** ladite ligne (L1) d'introduction de dialysat est dotée d'un élément de stockage flexible apte à stocker le dialysat circulant dans ladite ligne (L1) d'introduction de dialysat ; et **en ce que**, dans le processus de correction, le dialysat dans ladite poche (B1) de stockage de dialysat est permis d'être stocké dans l'élément de stockage.

6. Appareil de purification de sang selon la revendication 5, **caractérisé en ce que**, dans le processus de correction, ladite unité de commande (7) est configurée pour provoquer que le liquide dans l'élément de stockage est déchargé avant d'activer à tourner la pompe (P2) à dialysat, et elle est configurée pour assurer une capacité pour stocker le liquide qui est à circuler lors d'une activation de là la pompe (P2) à dialysat.

7. Appareil de purification de sang selon la revendication 5 ou 6, **caractérisé en ce que** ledit élément de stockage est une poche de chauffage (6) pour chauffer le dialysat circulant dans ladite ligne (L1) d'introduction de dialysat dans un traitement de purification de sang.

8. Appareil de purification de sang selon l'une quelconque des revendications 1 à 7, **caractérisé en ce que** le processus de correction est effectué avant un traitement de purification de sang.

9. Appareil de purification de sang selon l'une quelconque des revendications 1 à 7, **caractérisé en ce que** le processus de correction est effectué pendant un traitement de purification de sang.

10. Appareil de purification de sang selon l'une quelconque des revendications 1 à 9, **caractérisé en ce que**, dans le processus de correction, ladite unité de commande est destinée à corriger et commander la vitesse de rotation de la pompe (P2) à dialysat en fonction d'une erreur entre une valeur mesurée par ladite machine de pesage (4) est une valeur théorique.

11. Appareil de purification de sang selon l'une quelconque des revendications 1 à 10, **caractérisé en ce que**, dans le processus de correction, ladite unité de commande est configuré pour activer la pompe (P2) à dialysat à une pluralité de vitesses de rotation et pour déterminer des valeurs de correction en correspondance avec les vitesses de rotation respectives de la pompe (P2) à dialysat en fonction des variations des valeurs mesurées par ladite machine de pesage (4) obtenues aux vitesses de rotation respectives.

12. Appareil de purification de sang selon l'une quelconque des revendications 1 à 10, **caractérisé en ce que** les caractéristiques du taux de variation du débit par rapport à une nombre total des révolutions de la pompe (P2) à dialysat sont stockées préalablement ; et **en ce que**, dans le processus de correction, ladite unité de commande est configuré pour déterminer une valeur de correction en correspondance avec la vitesse de rotation de la pompe (P2) à dialysat en fonction de ces caractéristiques.
